(19) Europäisches Patentamt
European Patent Office
Office européen des brevets

(11) Publication number: **0 044 167**
**A2**

(12) # EUROPEAN PATENT APPLICATION

(21) Application number: 81302967.5

(22) Date of filing: 30.06.81

(51) Int. Cl.[3]: **A 61 K 39/395**
A 61 K 35/74, A 61 K 35/78

(30) Priority: 14.07.80 US 167863

(43) Date of publication of application:
20.01.82 Bulletin 82/3

(84) Designated Contracting States:
AT BE CH DE FR GB IT LI LU NL SE

(71) Applicant: THE REGENTS OF THE UNIVERSITY OF CALIFORNIA
2200 University Avenue
Berkeley, California 94720(US)

(72) Inventor: Collier, Robert John
650 Radcliffe Avenue
Pacific Palisades California 90272(US)

(72) Inventor: Gilliland, Dwight Gary
1634 Federal Avenue No. 5
Los Angeles California 90025(US)

(74) Representative: Harrison, David Christopher et al,
MEWBURN ELLIS & CO 70 & 72 Chancery Lane
London WC2A 1AD(GB)

(54) **Antibody targeted cytotoxic agent.**

(57) Specific cytotoxic agents are provided comprising antibody specific for a surface antigen joined to a cytotoxic agent, desirably by a reductively cleavable linkage. Particularly, the cytotoxic portion of various toxins may be joined to a monoclonal antibody or fragment thereof by a disulfide linkage. The conjugate is found to be toxic for susceptible cells having the determinant site recognized by the receptor. The conjugates can be used for isolating mutant culture cells and therapeutically for destroying mutant cells present in a host.

EP 0 044 167 A2

Croydon Printing Company Ltd

# ANTIBODY TARGETED CYTOTOXIC AGENT

The ability to kill specific eukaryotic cells which can be distinguished by a unique determinant or antigenic site offers many opportunities in research, as well as therapeutics. Cells are distinguishable by their surface antigens. Antibodies can be prepared which are specific for one or more unique determinant sites on a cell, so as to be able to distinguish the particular cell from different strains as well as species of cells. Where a cell mutates resulting in a change in the surface antigenicity, a distinctive marker is provided for recognizing the mutant cell.

A number of cytotoxic agents are known which are obtained from microorganisms or plants.

Efforts to use antibodies against specific cell-surface antigens to selectively kill populations of mammalian cells which express these antigens include conjugation of such cytotoxic agents as chlorambucil (Ghose et al., Cancer 29, 1398-1400, 1972, and Ghose et al., Br. Med. J. 3, 495-499, 1972), $^{131}I$ (Ghose et al., Cancer 36, 1646-1657, 1975); and whole diphtheria toxin (Moolten et al., J. Natl. Cancer Inst. 49, 1059-1062, 1972; Moolten et al., ibid 55, 473-477, 1975; and Thorpe et al., Nature 271 752-755 (1978)) to cell surface directed antibody. Oeltmann et al., J. Biol. Chem. 254, 1022-1027 (1979); and Oeltmann et al., ibid, 254, 1028-1032 (1979) disclose the A chain of ricin toxin linked to the B subunit of human chorionic gonadotropin.

In the invention, synthetic cell-specific cytotoxic agents are provided by coupling a non-specific cytotoxic agent to a cell-specific ligand via a linkage which is cleavable intracellularly, e.g. a disulfide bridge. Specifically, toxin A chains or analogous effector moieties are joined to surrogate binding moieties, particularly cell-surface directed antibodies, by disulfide linkages to provide cell specific toxic agents. Upon combining the toxic agents with cells having the specific determinant site, cell death rapidly follows, while the toxic agents have little effect on cells lacking the specific determinant site. The cell specific agents find use *in vitro* and *in vivo* in freeing cell cultures and tissues of specific cells.

Cytotoxic agents are provided by combining fragments of protein toxins which lack the toxin's intrinsic binding moiety with ligands which bind to specific receptors on the surface of cells. The cytotoxic agent compositions employed in the subject invention involve the toxin fragment, a binding moiety, and the linking group between the two portions.

The toxin fragment is a proteinaceous fragment from a naturally occurring toxin, which is substantially incapable of independent toxic activity, except when joined to its natural binding moiety or to a cell specific binding moiety other than the natural one. Many of the toxin fragments (termed "A" chains for activity) are enzymes that catalyze reactions within susceptible cells which inactivate specific components of the protein synthesis machinery within the cytosolic compartment. The toxic A chains are usually above 10,000 daltons in molecular weight, and may have nonproteinaceous side-groups, such as lipid or saccharides.

The proteinaceous cytotoxic agents can be further characterized by having substantially no cytotoxic activity (not greater than about $10^{-3}$ activity of the parent toxin as measured by protein synthesis inhibition) unless attached to a binding moiety, are capable of gaining access to the cytoplasm, can be covalently linked to a surrogate binding moiety, particularly an antibody or fragment thereof, and can be capable of acting as a cytotoxic agent, particularly as an enzyme catalyzing a reaction lethal to eukaryotic cells, and can kill virtually all cells once entry into the cytosol has occurred.

Illustrative toxin A chains or similar effector moieties include diphtheria toxin A chains, non-toxic enzymically active proteolytic fragments from Pseudomonas aeruginosa exotoxin A; ricin toxin A chain; abrin A chain; modeccin A chain; and proteins with activities similar to ricin, abrin and modeccin A chains found in various plants e.g. Gelonium multiflorum, Phytolacca americana, Croton tiglium, Jatropha curcas, Momordic charantia, and wheat germ. Also mutants species of the toxins may be used such as CRM45 (Boquet et al. (1976) PNAS USA 73, 4449-4453).

The diphtheria toxin A chain is known to catalyze the reaction of NAD with elongation factor-2, while the ricin A chain is known to inactivate the 60S ribosomal subunit.

The cell-surface binding moieties which are employed are monospecific in that they will be able to distinguish between various cells which may be present in a culture or host. Particularly, monoclonal antibodies, or specific binding fragments thereof, such as F(ab), $F(ab')_2$, or the like may be used.

The monoclonal antibodies are obtained from hybridomas. The manner of immunization, crossing of spleen cells with myeloma cells to produce hybridoma cells, and the production of monoclonal antibodies from the hybridoma cells has been extensively reviewed in the literature. See for example Koprowski et al. (1979) Somatic Cell Genetics 5, 957-972. The resulting monoclonal antibodies may be treated in a variety of ways, for example, papain digestion in a mild reducing environment to provide F(ab) fragments; or with pepsin to provide $F(ab')_2$ fragments. In each case, the significant factor is that the antibody of fragment thereof is specific for a particular determinant site which allows for distinguishing between the cells to be killed and other

BAD ORIGINAL

cells which may be present, which may share a large number or substantially all of the same determinant sites with the target cells. Since, for the most part, naturally occurring binding moieties compositions (monoclonal antibody compositions not being naturally available) will not be able to distinguish between target cells and closely similar cells by virtue of their natural binding sites, monoclonal antibodies and derivatives thereof will be the binding moiety of choice.

Finally, the toxic agent and the receptor will be linked, usually by a bond which is cleavable cytoplasmically. For the most part, the most convenient linkage employs a disulfide, particularly with one of the sulfurs intrinsic to the toxic agent. The disulfide may be joined to the two components in a variety of ways. Various functional groups include amino groups, carboxylic acid groups, imines, aldehydes, isocyanates, mercaptans and olefins, or the like, where the resulting linkage results in amides, imides, amidines, ureas, imines (Schiff's bases) thioethers or the like. The linkage group can be varied widely, since in many instances the toxin, as well as the receptor, may have mercaptides which may be used as the linkage. The portion extending from the disulfide binding to the toxic agent and joining the toxic agent to the receptor is not particularly significant and may be varied widely.

For the most part, the cytotoxic agents will have the following formula:

$$(AS_n)-(S-X-R)_m$$

wherein:

$AS_n$ indicates the toxic agent having one or more sulfur groups as part of the agent; n is 1 to the number of sulfur groups present in the toxic agent which are present as available mercaptide groups, generally being up to about 4; R is a monoclonal antibody receptor or derivative thereof; and m is 1 up to n, usually being from 1 to 2; and X is a linking group and may be a bond or a group of from about 1 to 20, usually 1 to 12 atoms other than hydrogen, which include carbon, nitrogen, oxygen and sulfur. Sulfur will normally be bonded to carbon, particularly aliphatically saturated. X

may be aliphatic, alicyclic, aromatic, heterocyclic or combinations thereof, generally having from 0 to 6, more usually from about 0 to 4, preferably about 1 to 4 heteroatoms, wherein oxygen and sulfur are present as oxo or non-oxo-carbonyl or the thio analogs thereof, or ether (including thioether), and nitrogen is present as amino or amido. For the most part the heteroatoms will be bonded solely to carbon.

Illustrative groups linking the disulfide include aminoethylene 3-propanyl methylene carbonyl, α-succinimidyl, 3-propylenethiocarbonyl. The groups which may be used are for the most part conventional groups providing for a disulfide linkage by having a disulfide compound capable of reacting with the cell-specific ligand, usually by amide formation, whereby a mercaptide group may be displaced from the disulfide resulting in a new disulfide linkage between the toxic agent and the ligand.

For the most part, the linkages will be aliphatic of from about 1 to 6 carbon atoms, providing for an amide bond to the receptor, although this is primarily a matter of convenience and not necessary to the operability of the subject compositions.

The compounds of the subject invention find use *in vitro* and *in vivo*. Where mixtures of cells are involved having different surface antigens, the subject invention provides for selective killing of cells having a specific surface antigen.

The cytotoxic effect of the compounds of the subject invention can be achieved in nutrient media, on nutrient agar, with a cell confluent layer, or other convenient environment involving cell growth or cell maintenance. Normally, there will be a multiplicity of at least one (toxin A chain)-(cell-surface ligand) conjugate per cell, more usually at least about 10, and there may be 100 or more. Preferably, there will be a significant excess of molecules of conjugates to numbers of cells. No particular manner of administration of the toxin conjugates to the cells is required, so long as

the administration involves contact between the conjugate and the cell.

For *in vivo* use, the conjugate may be administered parenterally or by injection, particularly intravenously. The amount of the conjugate employed will vary widely, depending upon the nature of the cell which is to be killed, the extent of the cell population, the resistance of the cell to the conjugate, the effectiveness of the conjugate, and the like. When administered at other than a specific site, usually, the amount of protein will vary from about 1μg to 10mg, usually up to 2mg, per kilogram of host weight. At a specific site, the amount of protein will be in the lower portion of the concentration range. The toxin A chain conjugate can be administered in a physiologically acceptable medium, such as phosphate buffered saline, saline, or other convenient vehicle. As powders, the conjugate may be compounded with other materials which may provide for directing the composition to a particular organ, protracted release, or the like. The manner in which proteinaceous compositions may be administered to a host finds ample exemplification in the prior art and will not be expounded upon here.

Of particular interest as target cells are tumor cells and pathogenic microorganisms. Of particular interest because of their widespread occurrence are lung carcinomas; colon and rectal cancer cells; female breast cancer; uterine carcinoma; prostatic carcinoma; bladder and kidney cancer; lymphoma; leukemia; and Hodgkins disease.

Illustrative of pathogenic microorganisms are protozoa, such as *Plasmodium vivas*, *P. maleriae*, *P. ovale*, and *P. falciparum*.

The following examples are offered by way of illustration and not by way of limitation.

# EXPERIMENTAL

## MATERIALS AND METHODS

### Cell Lines

Human cell lines. Eight different cell lines were used. SW 1116, SW 948, and SW 1083 are colorectal carcinoma (CRC) cell lines obtained from A. Leibovitz of the Scott and White Clinic, Temple, Texas. Lung carcinoma line MBA-9812 and normal lung fibroblast line HS-0853 were obtained from the Cell Culture Laboratory, Naval Biosciences Laboratory, Oakland, California. The MRC-5 line of normal fibroblasts was from Flow Laboratories, Rockville, Maryland. Melanoma cell lines WM 9 and WM 56 were established at the Wistar Institute.

Mouse cell lines. BALB/c myeloma P3x63Ag8 cells (abbreviated P3) were obtained from C. Milstein.

Hybridoma cell line. Hybridoma cell line 1083-17-1A (hereafter designated 17-1A) produces colorectal carcinoma-specific antibodies, and has been characterized previously (Herlyn et al. PNAS USA 76, 1438-1442; Koprowski et al., Somatic Cell Genetics 5, 957-972). Ascitic fluid was produced by inoculation of BALB/c mice i.p. with $10^7$ hybridoma 17-1A cells. The fluid was collected from mice which developed tumors after 2-3 weeks, centrifuged at 300 X g, and the supernatant was stored at -20°C. Purified 17-1A antibody was prepared from ascitic fluid with Staph A protein-Sepharose.

Fragment A (DTA) was prepared from diphtheria toxin (Connaught Laboratories) as described (Chung and Collier (1977) Biochem. Biophys. Acta 483, 248-257) and was heated to 80°C for 10 minutes to inactivate any residual traces of toxin. Enzymic activity of DTA was assayed by ADP-ribosylation of wheat germ elogation factor 2 with $^{14}C$-NAD as substrate. Ricin toxin was purified from castor beans (A. H. Hummert Seed Co., St. Louis, Missouri) by affinity chromatography on Sepharose 4B. (Nicolson and Blaustein (1972) ibid 266, 543-54 and modified by Cawley et al. Arch. Biochem. Biophys. 190, 744-755). Ricin toxin A-chain (RTA) was purified from whole ricin toxin by reduction from

2-mercaptoethanol and chromatography on Cellex-D (Bio-Rad) (Olsnes and Pihl, Biochem. 12, 3121-3126). RTA was freed of residual traces of ricin toxin by repeated cycling on Sepharose 4B colony.

Radioimmunoassay (RIA) was performed on live target cells at approximately 5 X $10^5$ cells per well, as described for melanoma cells (Koprowski et al. (1978) PNAS USA 75, 3405-3409). Target cells were incubated with the conjugates or control proteins for 1hr at room temperature. The cells were washed three times and then incubated with radiolabeled ($^{125}I$) rabbit antimouse $F(ab')_2$. After three final washes, radioactivity in the cell pellet was determined by counting in a Packard gamma spectrometer.

Cytotoxicity was measured by inhibition of protein synthesis as described previously (Gilliland et al. (1978) PNAS USA 75, 5319-5323). Briefly, 5 X $10^4$ cells in one ml of medium were plated in 8ml flint glass vials (ICN), incubated for 24hr, and then treated with an appropriate concentration of a conjugate or control protein. After an additional 24hr incubation, the medium was aspirated and replaced with a low amino acid medium (0.5ml) containing 0.2μCi per ml $^{14}C$-amino acid mixture.

N-succinimidyl-3-(2-pyridyldithio)propionate (SPDP) was from Pharmacia, dithiothreitol was from Vega Biochemicals, and all other reagents were as described (Gilliland, supra).

Synthesis of (DTA)-SS-(17-1A) antibody conjugates. 17-1A Antibody (7.0ml, 2mg/ml) was dialyzed against Dulbecco's phosphate buffered saline (DPBS Gibco), pH7.4, containing antibiotic-antimycotic solution (Gibco, 5.0ml per liter). SPDP (0.186ml, 20mM) in absolute ethanol was added to the antibody with vigorous mixing. The mixture was allowed to react for 30 minutes at room temperature and then dialyzed against two 1 liter changes of the same buffer. After dialysis the antibody preparation was analyzed for 2-pyridyldisulfide content as described (Stuchbury et al. (1975) Biochem. J. 151, 417-432), and found to contain 4.3 such residues per antibody molecule. DTA (3.0ml, 2.5mg/ml)

was reduced by addition of 0.3ml of 1.0M dithiothreitol, pH7.0, for 30min at room temperature and desalted on Sephadex G-25 (2.6 X 12 cm column) equilibrated with the buffer described above. Peak fractions from the column were pooled (11.0ml, 0.53mg/ml) and mixed with PDP-(17-1A) antibody (7.0ml, 2.1mg/ml). Final concentrations of DTA and 17-1A antibody were 1.5 X $10^{-5}$M and 5.5 X $10^{-6}$M, respectively. The final molar ratio of DTA to 17-1A antibody in the reaction mixture was 2.8. The crude conjugate preparation (18.0ml) was concentrated to a final volume of 9.0ml by ultracentrifugation on an Amicon YM-10 membrane. Crude (DTA)-SS-(17-1A) (9.0ml) was chromatographed on a Sephacryl S-200 column (2.6 X 106cm, 22.1ml/h flow rate) equilibrated with DPBS buffer. Each fraction (6.2ml) was analyzed for ADP-ribosylation activity and by $NaDodSO_4$/polyacrylamide gel electrophoresis. Fractions 30-40 were pooled, concentrated on an Amicon YM-10 membrane, and used in cytotoxicity assays after filter sterilization.

<u>Synthesis of (RTA)-SS-(17-1A) antibody conjugate</u>. (RTA)-SS-(17-1A) was synthesized as described for (DTA)-SS-(17-1A) with the following modifications: 17-1A antibody (4ml, 2.5mg/ml) was reacted with SPDP (0.167ml, 20mM in absolute ethanol) to give 8.9mol PDP per mol antibody. RTA (7.0ml, 1.1mg/ml) was reduced, desalted, and mixed with PDP-(17-1A) to give a final concentration of 1.2 X $10^{-5}$M and 3.9 X $10^{-6}$M, for RTA and 17-1A, respectively. RTA was in 3.1 fold molar excess to antibody in the reaction mixture. The crude conjugate mixture was concentrated to 10ml on an Amicon YM-10 membrane before chromatography on Sephacryl S-200, as described above.

<u>Synthesis of (DTA)-SS-(17-1A) antibody conjugate using cystamine</u>. The procedure has been described previously by Gilliland et al. PNAS USA <u>75</u>, 5319-5323 (1978). All steps are performed at 4°C or on ice. 17-1A Antibody (5.1mg/ml, 2ml) is applied to a 2.4 x 9 cm Sephadex G25 column equilibrated with 0.2<u>M</u> NaCl, 0.02% $NaN_3$ and 1μg phenylmethylsulfonylfluoride per ml. Peak fractions are pooled 14.0ml, 2.4mg/ml, mixed with cystamine hydrochloride (45mg) and

adjusted to pH 4.7 by addition of 0.1N HCl. Ethyl dimethylaminopropyl carbodiimide is added to a final concentration of 7.5 mg/ml and the reaction mixture maintained at pH 4.7 for 30min.

After addition of 1M NaOAc (0.2ml), the preparation is dialyzed exhaustively against Buffer A. The resulting cystaminyl-(17-1A) antibody conjugate (1mg/ml, 6ml) is reduced by addition of 0.2ml 1M dithiothreitol, pH7.0, and incubation for 1hr at rt, followed by desalting on a Sephadex G-25 column (2.6 x 11 cm) equilibrated with Buffer A. Peak fractions are pooled and mixed with cystaminyl-(17-1A) (3.0ml, 2.4mg/ml). Final concentrations of ($^{125}$I-DT-A) and cystaminyl-(17-1A) are 1.6 x $10^{-5}$M and 2.6 x $10^{-6}$M respectively in a total volume of 18ml. Nearly all of the cystaminyl-(17-1A) antibody is converted to higher molecular weight products as evidenced by autoradiography. Crude conjugate is concentrated to a final volume of 9ml on an Amicon YM-10 membrane and purified on a Sephacryl S-200 column (4.5 x 42cm) equilibrated with Buffer A and fractions (2.0ml) collected at a rate of 13.1ml/hr.

Specific binding of (DTA)-SS-(17-1A) and (RTA)-SS-17-1A) conjugates to target cells. Binding of the A-chain conjugates to various cell lines was assayed to insure that modified 17-1A antibody retained activity and specificity for colorectal carcinoma cells. Cells were first incubated with a conjugate or control protein. The cells were then washed and incubated with $^{125}$I-antibody directed against the F(ab) region of the 17-1A antibody, and cell-bound radioactivity was measured. (DTA)-SS-(17-1A), (RTA)-SS-(17-1A), and unmodified 17-1A antibody each bound to colorectal carcinoma cell lines SW 1116 and SW 948. As expected, no binding was detected if P3 antibody (that from the parental myeloma) or either of the unconjugated A-chains was added to cells. Unmodified 17-1A antibody apparently bound more efficiently than (RTA)-SS-(17-1A), which in turn bound to a greater extent than (DTA)-SS-(17-1A). This difference may result from steric hindrance either of the binding of $^{125}$I-anti-$F(ab')_2$ to DTA- or RTA-modified 17-1A antibody, or hindrance

of 17-1A binding to the cell surface because of the attached A-chain, or both causes.

Specificity was demonstrated by measuring conjugate binding to cell lines which lack antigen. (DTA)-SS-(17-1A) bound to both SW 948 and SW 1116, but did not bind to four other cell lines which do not express antigen: MRC-5 (human embryo fibroblast), WM 56 (melanoma), MBA 9812 (lung carcinoma), and HS-0853 (normal lung fibroblast). These results correlate with the known binding specificity of 17-1A antibody. The higher level of binding of the conjugate to SW 948 cells relative to SW 1116 cells reflects a difference in the number of cells in the assay, not in the amount of antigen expressed by SW 948 cells.

Cytotoxicity of conjugates for colorectal carcinoma cells. Cytotoxicity of (DTA)-SS-17-1A) and (RTA)-SS-(17-1A) for colorectal carcinoma cells was tested by a protein synthesis inhibition assay described previously (Gilliland et al. (1978) PNAS USA 75, 5319-5323). Briefly, cells were assayed for ability to incorporate $^{14}C$-amino acids into trichloroacetic acid-precipitable material after a 24hr exposure to conjugate or control protein. Both (DTA)-SS-(17-1A) and (RTA)-SS-(17-1A) were toxic for SW 1116 cells, giving 50% inhibition at a concentration of about $10^{-9}$M. (DTA)-SS-(17-1A) and (RTA)-SS-(17-1A) were at least 100-fold more toxic than controls, which included unconjugated DTA, RTA, and 17-1A antibody. The A-chain conjugates were non-toxic for melanoma cells (WM 56) at concentrations as high as $10^{-7}$M, although these cells were as sensitive as SW 1116 cells to whole diphtheria toxin. Cytotoxicity was therefore dependent on the presence of cell surface antigen capable of binding 17-1A monoclonal antibody.

Further evidence of specificity of the conjugates for cells which contain antigen is summarized in the following Table 1.

TABLE 1

PERCENT OF PROTEIN SYNTHESIS INHIBITION BY CONJUGATES

| CELLS | | DTA-SS-(1083-17-1A) | | RTA-SS (1083-17-1A) | |
|---|---|---|---|---|---|
| ORIGIN | LINE | $10^{-8}$* | $10^{-7}$ | $10^{-8}$ | $10^{-7}$ |
| Colo-rectal Carcinoma | SW 948<br>SW 1116 | 80<br>87 | 96<br>96 | 81<br>92 | 94<br>96 |
| Lung Carcinoma | MBA 9812 | 7 | 27 | 2 | 0 |
| Mela-noma | WM 9<br>WM 56 | 6<br>11 | 3<br>0 | 0<br>0 | 8<br>8 |
| Embryonal Fibro-blasts | MRC 5 | 0 | 1 | 0 | 0 |
| Normal Lung Fibro-blasts | HS 0853 | 0 | 0 | 0 | 0 |

* = Molar concentration of conjugate.

The conjugates were not toxic for lung carcinoma, melanoma, human embryo fibroblast and normal lung fibroblast cell lines at concentrations at which nearly 100% inhibition of protein synthesis was observed in colorectal carcinoma cell lines SW 948 and SW 1116. Specificity of the toxic activity correlated with the binding specificity of the conjugates and unmodified 17-1A antibody for these cell lines.

These results imply that it is possible to specify conditions where most, if not all, cells expressing antigen are killed, but cells lacking antigens are unaffected. This was effectively shown in the plating efficiency experiment described in the following Table 2.

TABLE 2

PLATING EFFICIENCY

DTA-SS-17-1A

| CELL LINES | 0 | 50 ng/ml | |
|---|---|---|---|
| | CONTROL | DTA-SS-17-1A | SPDP-17-1A |
| SW 403 | 64 | 34 | 62 |
| SW 1083 | 36 | 13 | 28 |
| SW 1116 | 38 | 33 | 34 |
| WM 56 | 29 | 27 | 25 |

TABLE 2 - continued

| CELL LINES | 50 μg/ml | |
|---|---|---|
| | DTA-SS-17-1A | SPDP-17-1A |
| SW 403 | 0 | 34 |
| SW 1083 | 2 | 17 |
| SW 1116 | 0 | 36 |
| WM 56 | 24 | 22 |

Colorectal carcinoma (SW 403, SW 1083, SW 1116) and melanoma (WM 56) cells were inoculated in 24 well Linbro plates at the density of 100 cells per well. After overnight culture, the medium was removed and replaced with medium containing (DTA)-SS-(17-1A) conjugate or its intermediate SPDP-17-1A (2ml/well). Following 10 days of culture the colony number per well was calculated.

(DTA)-SS-(17-1A) at 50μg/ml killed virtually all colorectal carcinoma cells (SW 403, SW 1083, SW 1116) under conditions where the melanoma cell lines WM 56 was unaffected. The reason for nonspecific toxicity of the PDP-(17-1A)-antibody preparation for SW 403 and SW 1083 cells is not clear, but the preparation was nontoxic for melanoma cells, to which the antibodies do not bind.

To further demonstrate the effectiveness of the compositions of the subject invention, colorectal carcinoma

cells were mixed with the (DTA)-SS-(17-1A) or (RTA)-SS-(17-1A) conjugate or unconjugated 17-1A antibody and after 20min at room temperature, the mixtures were injected into nude mice (six mice per group); 200μg conjugate or antibody and 5 X $10^6$ cells per animal. After three weeks, all six animals in the control group (unconjugated antibody) showed tumors, whereas none of the animals receiving conjugate (0/6 and 0/6 for the two conjugates, respectively) showed evidence of tumor formation.

It is evident from the above results that the subject conjugates are efficient cytotoxic agents for target cells, without having significant cytotoxic effect against other cells which may only differ in minor ways as to surface antigens. The subject invention permits the preparation of cell specific receptors to provide the ability to kill specific target cells despite the presence of other cells which may only differ by one or more epitopes. The subject compositions can be introduced into a host and are found to be effective in protecting the host from cellular invasion.

Although the foregoing invention has been described in some detail by way of illustration and example for purposes of clarity of understanding, it will be obvious that certain changes and modifications may be practiced.

WHAT IS CLAIMED IS:

1. A composition consisting essentially of, as the active cytotoxic agent, a binding moiety monospecific for a cellular surface epitope of a susceptible cell joined to an enzymically-active fragment of a toxin by a linkage which is cleavable in the cytoplasm of susceptible cells.

2. A composition according to claim 1, wherein said binding moiety is monoclonal antibody.

3. A composition according to claim 1, wherein said binding moiety is a fragment of monoclonal antibody, or a crosslinked aggregate of monoclonal antibodies.

4. A composition according to claims 1, 2 or 3, wherein said linkage involves a disulfide bridge.

5. A composition according to claim 4, wherein said fragment is a diphtheria toxin A chain.

6. A composition according to claim 4, wherein said fragment is a ricin toxin A chain.

7. A method for inhibiting multiplication of a cell having a specific surface epitope in the presence of other cells lacking said specific surface epitope, which comprises:

treating the mixture of cells with a composition according to claim 1, wherein said binding moiety is specific for said surface epitope.

8. A method according to claim 7, wherein said cell having said specific epitope is a carcinoma cell.

9. A method of freeing a host of a target cell having a specific surface epitope which comprises:

administering to said host in an amount sufficient to be cytotoxic to said cell, a composition according to claim 1, wherein said binding moiety is specific for said surface epitope.

10. A method according to claim 9, wherein said cell is a tumor cell.

11. A method according to claim 10, wherein said tumor cell is a colorectal tumor cell.